# EUROPÄISCHE PATENTSCHRIFT

(11) **EP 0 978 503 B1**
(45) Veröffentlichungstag und Bekanntmachung des Hinweises auf die Patenterteilung: **07.05.2003**
(21) Anmeldenummer: 99114921.2
(22) Anmeldetag: 30.07.1999
(51) Int. Cl.: C07C 67/04

(54) **Synthese von o-(p-Tolyl)benzoesäure-tert.-butylester durch Umsetzung von o-(p-Tolyl)benzoesäure mit Isobutylen unter Säurekatalyse**
Synthesis of t-butyl o-(p-tolyl)benzoate by reaction of o-(p-tolyl)benzoic acid with isobutene using acid catalysis
Sythèse de t-butyl o-(p-tolyl)benzoate par réaction d'acide o-(p-tolyl)benzoique avec isobutène utilisant catalyse acide

(30) Priorität: 05.08.1998 DE 19835358
(43) Veröffentlichungstag der Anmeldung: 09.02.2000
(73) Patentinhaber: Clariant GmbH, 65929 Frankfurt am Main (DE)
(72) Erfinder: Meudt, Andreas, Dr., 65439 Flörsheim-Weilbach (DE); Nörenberg, Antje, Dr., 64572 Büttelborn (DE); Haber, Steffen, Dr., 76829 Landau/Pfalz (DE)

(56) Entgegenhaltungen:
- US-A- 4 921 999

## Beschreibung

Die Erfindung betrifft ein neues Verfahren zur Herstellung von o-(p-Tolyl)benzoesäure-tert.-butylester der nachstehenden Formel (I).

Ortho-(p-tolyl)benzoesäure-Derivate sind von großer Bedeutung als Syntheseintermediate von potenten, bei oraler Aufnahme wirksamen AT II-Antagonisten.

Aufgrund der Bedeutung von o-(p-Tolyl)benzoesäure-tert.-butylester sind eine Reihe von Synthesemöglichkeiten bekannt, beispielsweise a) Kupplung eines α-Halogenbenzoesäure-tert.-butylesters mit p-Tolylmetallaten und b) Umsetzung von o-(p-Tolyl)benzoesäure zum entsprechenden tert.-Butylester. Bei erstgenannter Variante muß man zunächst das entsprechende p-Tolylmetallat, beispielsweise p-Toluolboronsäure, herstellen, um dieses dann mit o-Halogenbenzoesäure-tert.butylestern umzusetzen, die ebenfalls erst aus den entsprechenden o-Halogenbenzoesäuren dargestellt werden müssen. Die als Edukt in Variante b) eingesetzte o-(p-Tolyl)benzoesäure ist hingegen kommerziell erhältlich.

Methoden zur Herstellung von tert.-Butylestern ausgehend von entsprechenden Carbonsäuren, sind beispielsweise beschrieben in A.L. McCloskey, G.S. Fonken, Org. Synth. Collect, Vol. IV, 261, weisen jedoch die Nachteile einer Mehrstufensynthese, dadurch bedingte mäßige Gesamtausbeuten und die Verwendung korrosiver und wasserempfindlicher Reagentien oder Zwischenstufen auf, beispielsweise die Verwendung von Säurechloriden und kalium-tert.-butanolat.

Ebenfalls bekannt ist die Umsetzung von Carbonsäuren mit Isobutylen unter der Katalyse einer starken Säure, meist Schwefelsäure. Allerdings muß man hierbei teilweise hohe Drücke und lange Reaktionszeiten anwenden, um akzeptable Umsätze zu gewährleisten (A.L. McCloskey, G.S. Fonken, Org. Synth. Collect, Vol. IV).

In der US-PS 4,921,999 werden Adamantan-1-carbonsäure und o-Benzoylbenzoesäure mit Isobutylen in Gegenwart von Trifluormethansulfonsäure verestert. Ausbeute und Reinheit sind jedoch unbefriedigend.

Der Erfindung lag die Aufgabe zugrunde, ein industriell durchführbares Verfahren bereitzustellen, das einen verfahrenstechnisch einfachen und kostengünstigen Zugang zu o-(p-Tolyl)benzoesäure-tert.-butylester in hoher Reinheit und in guten Ausbeuten liefert.

Überraschenderweise wurde gefunden, daß o-(p-Tolyl)benzoesäure-tert.-butylester der Formel (I) in einfacher Weise durch Umsetzung von o-(p-Tolyl)benzoesäure (II) mit Isobutylen (III) unter Katalyse bestimmter Protonsäuren in sehr hohen Reinheiten und in nahezu quantitativer Ausbeute zugänglich ist:

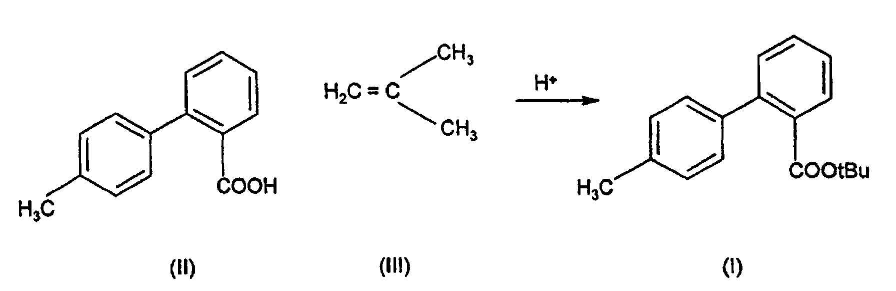

Gegenstand der vorliegenden Erfindung ist daher ein Verfahren zur Herstellung von o-(p-Tolyl)benzoesäure-tert.-butylester, dadurch gekennzeichnet, daß man o-(p-Tolyl)benzoesäure mit Isobutylen in Gegenwart einer katalytisch wirksamen Menge an Fluorsulfonsäure, Trifluormethansulfonsäure, einer geradkettigen oder verzweigten Perfluor-(C₂-C₈)alkylsulfonsäure, einer geradkettigen oder verzweigten teilfluorierten (C₂-C₈)Alkylsulfonsäure, einer teilfluorierten (C₅-C₆)-Cycloalkylsulfonsäure oder einer Mischung dieser Säuren umsetzt.

Als Perfluor-(C₂-C₈)alkylsulfonsäuren kommen insbesondere C₂F₅SO₃H, C₃F₇SO₃H, C₄F₉SO₃H und C₈F₁₇SO₃H in Betracht. Als teilfluorierte (C₂-C₈)Alkylsulfonsäuren kommen insbesondere CF₃-CHF-CF₂-SO₃H, CF₂H-CF₂-SO₃H und n-C₅F₁₁-CHF-CF₂-SO₃H in Betracht. Als teilfluorierte (C₅-C₆)Cycloalkylsulfonsäure kommt insbesondere 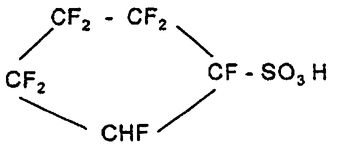 in Betracht.

Die als Katalysator eingesetzte Säure wird zweckmäßigerweise in einer Menge von 0,001 bis 10 mol-%, vorzugsweise 0,1 bis 4 mol-%, bezogen auf o-(p-Tolyl)-benzoesäure, eingesetzt.

Die Reaktion wird zweckmäßigerweise bei Temperaturen zwischen -40°C und +7°C durchgeführt, bevorzugt zwischen -30°C und +5°C, besonders bevorzugt zwischen -15 und +2°C.

Als Lösungsmittel können aprotische, aromatische oder aliphatische Lösungsmittel verwendet werden, z.B. halogensubstituierte Benzole, Toluol, Nitrobenzol oder Neopentan, bevorzugt Chlorbenzol oder o-Dichlorbenzol. Die Menge an Lösungsmittel kann in weiten Grenzen schwanken, zweckmäßig sind jedoch 20 bis 1000, vorzugsweise 100 bis 800, insbesondere 150 bis 650, Gewichtsteile Lösungsmittel je 100 Gewichtsteile o-(p-Tolyl)-benzoesäure.

Die molaren Verhältnisse zwischen o-(p-Tolyl)benzoesäure und Isobutylen betragen zweckmäßigerweise 1:1 bis 1:100, bevorzugt 1:2 bis 1:10. Die erfindungsgemäße Reaktion kann bei Normaldruck durchgeführt werden, führt aber auch bei höheren Drücken zu quantitativen Ausbeuten an hochreinem Produkt.

Die Reaktion wird vorteilhaft in der Weise durchgeführt, daß man eine Lösung von o-(p-Tolyl)benzoesäure im gewählten Lösungsmittel bei der gewünschten Temperatur vorlegt, den Katalysator hinzufügt und flüssiges oder gasförmiges Isobutylen zudosiert.

Nach Beendigung der Umsetzung wird eine Base zugegeben, vorzugsweise wäßrige Natronlauge, organische und wäßrige Phase getrennt und das gewünschte Produkt isoliert.

Die folgenden Beispiele sollen die Erfindung erläutern, ohne sie zu begrenzen.

### Beispiel 1

Bei einer Temperatur von -10°C wurden 10,6 g 2-(p-Tolyl)-benzoesäure (50 mmol) und 100 mg Fluorsulfonsäure (1 mmol; 2 mol-%) in 60 ml Chlorbenzol vorgelegt. Im Laufe von 5 Minuten wurden insgesamt 12 ml flüssiges Isobutylen zugetropft. Nach 60 minütigem Rühren wurde die Reaktion durch Zugabe von 20 ml 10 gew.-%ige NaOH gestoppt. Nach Phasentrennung und Abdestillieren des Lösungsmittels wurde 2-(p-Tolyl)benzoesäure-t-butylester als farblose Flüssigkeit in einer Ausbeute und Reinheit von jeweils > 99 % (HPLC/UV und GC/FID) erhalten.

### Beispiel 2

Bei einer Temperatur von 0°C wurden 10,6 g 2-(p-Tolyl)benzoesäure und 150 mg Nonafluor-n-butansulfonsäure (0,5 mmol, 1 mol-%) in 60 ml Chlorbenzol vorgelegt. Im Laufe von 5 Minuten wurden insgesamt 12 ml Isobutylen einkondensiert. Nach 60 minütigem Rühren wurde die Reaktion durch Zugabe von 20 ml 10 gew.-%iger NaOH gestoppt. Nach der gleichen Aufarbeitung wie in Beispiel 1 wurde 2-(p-Tolyl)benzoesäure-t-butylester als farblose Flüssigkeit in einer Ausbeute und Reinheit von jeweils > 99 % (HPLC/UV und GC/FID) erhalten.

### Beispiel 3

Bei einer Temperatur von 0°C wurden 10,6 g 2-(p-Tolyl)benzoesäure und 7,5 mg Trifluormethansulfonsäure (0,1 mol-%) in 30 ml Chlorbenzol vorgelegt. Im Laufe von 5 Minuten wurden insgesamt 12 ml Isobutylen einkondensiert. Nach 60 minütigem Rühren wurde die Reaktion durch Zugabe von 20 ml 10 gew.-%iger NaOH gestoppt. Nach der gleichen Aufarbeitung wie in Beispiel 1 wurde 2-(p-Tolyl)benzoesäure-tbutylester als farblose Flüssigkeit in einer Ausbeute und Reinheit von jeweils > 99 % (HPLC/UV und GC/FID) erhalten.

## Patentansprüche

1. Verfahren zur Herstellung von o-(p-Tolyl)benzoesäure-tert.-butylester der Formel (I) 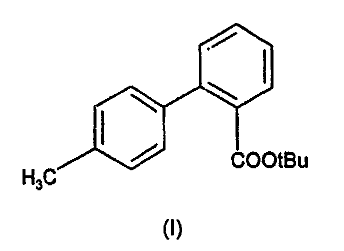 **dadurch gekennzeichnet, daß** man o-(p-Tolyl)benzoesäure mit Isobutylen in Gegenwart einer katalytisch wirksamen Menge an Fluorsulfonsäure, Trifluormethansulfonsäure, einer geradkettigen oder verzweigten Perfluor-(C₂-C₈)alkylsulfonsäure, einer geradkettigen oder verzweigten teilfluorierten (C₂-C₈)Alkylsulfonsäure, einer teilfluorierten (C₅-C₆)-Cycloalkylsulfonsäure oder einer Mischung dieser Säuren umsetzt.

2. Verfahren nach Anspruch 1, **dadurch gekennzeichnet, daß** die als Katalysator eingesetzte Säure Fluorsulfonsäure, CF₃SO₃H, C₂F₅SO₃H, C₃F₇SO₃H, C₄F₉SO₃H, C₈F₁₇SO₃H, CF₃-CHF-CF₂-SO₃H, CF₂H-CF₂-SO₃H, n-C₅F₁₁CHF-CF₂-SO₃H oder 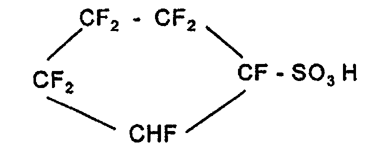 ist.

3. Verfahren nach Anspruch 1 oder 2, **dadurch gekennzeichnet, daß** die als Katalysator eingesetzte Säure in einer Menge von 0,001 bis 10 mol-%, vorzugsweise 0,1 bis 4 mol-%, bezogen auf o-(p-Tolyl)-benzoesäure eingesetzt ist.

4. Verfahren nach mindestens einem der Ansprüche 1 bis 3, **dadurch gekennzeichnet, daß** die Umsetzung bei einer Temperatur zwischen -40°C und +7°C, vorzugsweise zwischen -15°C und +2°C, durchgeführt wird.

5. Verfahren nach mindestens einem der Ansprüche 1 bis 4, **dadurch gekennzeichnet, daß** ein aprotisches aromatisches oder aliphatisches Lösungsmittel verwendet wird.

6. Verfahren nach mindestens einem der Ansprüche 1 bis 5, **dadurch gekennzeichnet, daß** ein halogensubstituiertes Benzol, Toluol, Nitrobenzol oder Neopentan als Lösungsmittel verwendet wird.

7. Verfahren nach mindestens einem der Ansprüche 1 bis 6, **dadurch gekennzeichnet, daß** Chlorbenzol oder o-Dichlorbenzol als Lösungsmittel verwendet wird.

8. Verfahren nach mindestens einem der Ansprüche 1 bis 7, **dadurch gekennzeichnet, daß** man eine Lösung von o-(p-Tolyl)-benzoesäure vorlegt, den Katalysator hinzufügt und flüssiges oder gasförmiges Isobutylen zudosiert.

9. Verfahren nach mindestens einem der Ansprüche 1 bis 8, **dadurch gekennzeichnet, daß** nach Beendigung der Umsetzung eine Base zugegeben wird.

## Claims

1. A process for the preparation of tert-butyl o-(p-tolyl)benzoate of the formula (I) 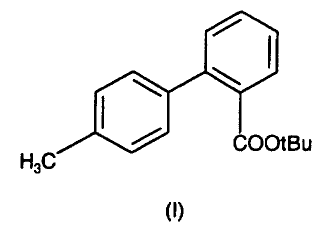 which comprises reacting o-(p-tolyl)benzoic acid with isobutylene in the presence of a catalytically active amount of fluorosulfonic acid, trifluoromethanesulfonic acid, a straight-chain or branched perfluoro-(C₂-C₈)alkylsulfonic acid, a straight-chain or branched partially fluorinated (C₂-C₈)alkylsulfonic acid, a partially fluorinated (C₅-C₆)-cycloalkylsulfonic acid or a mixture of these acids.

2. The process as claimed in claim 1, wherein the acid employed as a catalyst is fluorosulfonic acid, CF₃SO₃H, C₂F₅SO₃H, C₃F₇SO₃H, C₄F₉SO₃H, C₈F₁₇SO₃H, CF₃-CHF-CF₂-SO₃H, CF₂H-CF₂-SO₃H, n-C₅F₁₁CHF-CF₂-SO₃H or

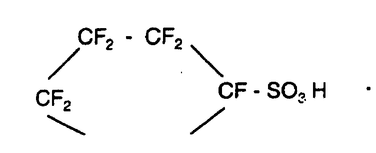

3. The process as claimed in claim 1 or 2, wherein the acid employed as a catalyst is employed in an amount from 0.001 to 10 mol%, preferably 0.1 to 4 mold, based on o-(p-tolyl)benzoic acid.

4. The process as claimed in at least one of claims 1 to 3, wherein the reaction is carried out at a temperature between -40°C and +7°C, preferably between -15°C and +2°C.

5. The process as claimed in at least one of claims 1 to 4, wherein an aprotic aromatic or aliphatic solvent is used.

6. The process as claimed in at least one of claims 1 to 5, wherein a halogen-substituted benzene, toluene, nitrobenzene or neopentane is used as a solvent.

7. The process as claimed in at least one of claims 1 to 6, wherein chlorobenzene or o-dichlorobenzene is used as a solvent.

8. The process as claimed in at least one of claims 1 to 7, wherein a solution of o-(p-tolyl)benzoic acid is introduced, the catalyst is added and liquid or gaseous isobutylene is metered in.

9. The process as claimed in at least one of claims 1 to 8, wherein a base is added after completion of the reaction.

## Revendications

1. Procédé pour la préparation d'o-(p-tolyl)benzoate de butyle de formule (I) 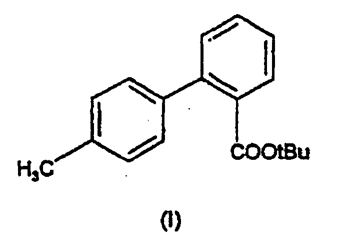 **caractérisé en ce qu'**on fait réagir l'acide o-(p-tolyl)benzoïque sur l'isobutylène en présence d'une quantité catalytique d'acide fluorosulfonique, d'acide trifluorométhanesulfonique, d'un acide perfluoro(alkyl en C₂-C₈)sulfonique partiellement fluoré linéaire ou ramifié, d'un acide (alkyl en C₂-C₈)sulfonique linéaire ou ramifié, d'un acide cyclo(alkyl en C₅-C₆)sulfonique partiellement fluoré ou un mélange de ces acides.

2. Procédé selon la revendication 1, **caractérisé en ce que** l'acide utilisé comme catalyseur est l'acide fluorosulfonique, CF₃SO₃H, C₂F₅SO₃H, C₃F₇SO₃H, C₄F₉SO₃H, C₈F₁₇SO₃H, CF₃-CHF-CF₂-SO₃H, CF₂H-CF₂-SO₃H, n-C₅F₁₁-CHF-CF₂-SO₃H ou

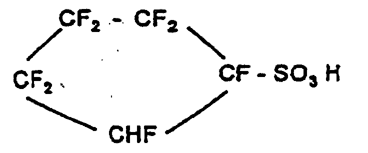

3. Procédé selon la revendication 1 ou 2, **caractérisé en ce qu'**on utilise l'acide employé comme catalyseur dans une quantité de 0,001 à 10 % molaires, de préférence de 0,1 à 4 % molaires, par rapport à l'acide o-(p-tolyl)benzoique.

4. Procédé selon au moins l'une des revendications 1 à 3, **caractérisé en ce qu'**on met en oeuvre la réaction à une température comprise entre -40°C et +7°C, de préférence entre -15°C et +2°C.

5. Procédé selon au moins l'une des revendications 1 à 4, **caractérisé en ce qu'**on utilise un solvant aprotique aromatique ou aliphatique.

6. Procédé selon au moins l'une des revendications 1 à 5, **caractérisé en ce qu'**on utilise en tant que solvant un benzène, un toluène, un nitrobenzène ou un néopentane substitué par un substituant halogène.

7. Procédé selon au moins l'une des revendications 1 à 6, **caractérisé en ce qu'**on utilise le chlorobenzène ou l'o-dichlorobenzène en tant que solvant.

8. Procédé selon au moins l'une des revendications 1 à 7, **caractérisé en ce qu'**on place une solution d'acide o-(p-tolyl)benzoique, on ajoute le catalyseur et on ajoute progressivement l'isobutylène liquide ou gazeux.

9. Procédé selon au moins l'une des revendications 1 à 8, **caractérisé en ce qu'**on ajoute une base après la fin de la réaction.
